(19) Europäisches Patentamt
European Patent Office
Office européen des brevets

(11) Publication number: **0 090 569 B1**

(12) **EUROPEAN PATENT SPECIFICATION**

(45) Date of publication of patent specification: **08.01.86**

(51) Int. Cl.[4]: **C 07 C 2/12**, B 01 J 29/18, C 07 C 11/08, C 07 C 7/177

(21) Application number: **83301545.6**

(22) Date of filing: **18.03.83**

(54) **A process for the low polymerization of isobutene.**

(30) Priority: **23.03.82 JP 44684/82**
**26.03.82 JP 47316/82**

(43) Date of publication of application:
**05.10.83 Bulletin 83/40**

(45) Publication of the grant of the patent:
**08.01.86 Bulletin 86/02**

(84) Designated Contracting States:
**DE FR GB IT NL**

(56) References cited:
**CHEMICAL ABSTRACTS, vol. 84, no. 25, 21st June 1976, page 522, no. 179631r, Columbus, Ohio, USA**

**CHEMICAL ABSTRACTS, vol. 97, jo. 26, 1982, page 14, no. 216913a, Columbus, Ohio, USA**

(73) Proprietor: **TOA NENRYO KOGYO KABUSHIKI KAISHA**
**1-1 Hitotsubashi, 1-Chome Chiyoda-Ku**
**Tokyo 100 (JP)**

(72) Inventor: **Sakurada, Satoshi**
**745-7, Sashioogi**
**Omiya-shi Saitama-ken (JP)**
Inventor: **Hashimoto, Takao**
**1902-5, Ooaza-Kamekubo Ooimachi**
**Iroma-gun Saitama-Ken (JP)**
Inventor: **Tagaya, Nobuaki**
**2735-4, Aza-Oogasa Kasahata**
**Kawagoe-shi Saitama-ken (JP)**
Inventor: **Maeshima, Tsugio**
**1428-4, Suneori Tsurugashima-cho**
**Iruma-gun Saitama-ken (JP)**
Inventor: **Ueda, Kayako**
**44-18, Shakujhiicho 8-chome**
**Nerima-ku Tokyo (JP)**
Inventor: **Kokubo, Masahiro**
**1902-5, Ooaza-Kamekubo Ooimachi**
**Iruma-gun Saitama-ken (JP)**

(74) Representative: **Allard, Susan Joyce et al**
**BOULT, WADE & TENNANT 27 Furnival Street**
**London EC4A 1PQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 090 569 B1

**Description**

This invention relates to a process for the low polymerization of isobutene using a solid acid catalyst, in particular, high silica mordenite catalyst with a specified solid acid quantity, and more particularly, it is concerned with a process for the selective low polymerization of only isobutene in an isobutene-containing hydrocarbon mixture.

In the low polymerization of isobutene in a hydrocarbon mixture containing isobutene, it is well known to contact the hydrocarbon mixture with a solid acid catalyst such as silica alumina, zeolite or a cation exchange resin. This method is used for the removal of isobutene from $C_4$ hydrocarbon mixtures.

For example, there have hitherto been proposed (1) a method comprising contacting a $C_4$ hydrocarbon mixture with an isomerization catalyst such as palladium, platinum or nickel to isomerize 1-butene to 2-butene, further contacting the mixture with a solid acid catalyst such as activated clay or silica alumina to polymerize isobutene and separating the resulting low polymers (Japanese Patent Application OPI (Kokai) No. 8201/1976), (2) a method comprising contacting a $C_4$ hydrocarbon mixture with a crystalline molecular sieve (10X Molecular Sieve) having effective pores of about 8 to 8.2Å to remove isobutene (Japanese Patent publication No. 42803/1979), (3) a method comprising contacting a $C_4$ hydrocarbon mixture with a synthetic zeolite (ZSM-4) to selectively polymerize isobutene (Japanese Patent Publication No. 29121/1979) and (4) a method comprising feeding a $C_4$ hydrocarbon mixture to a distillation column packed with a cation exchange resin, polymerizing the isobutene and separating and removing the low polymers from the bottom thereof (US Patent No. 4,215,011).

However, none of these methods is suitable as a method of producing 1-butene free from isobutene since the 1-butene contained in the $C_4$ hydrocarbon mixture tends to be isomerized to 2-butene.

We have now developed an improved process for the low polymerization of isobutene using a solid acid catalyst and for selectively polymerizing isobutene contained in 1-butene as an impurity and a method of purifying 1-butene containing isobutene.

We have also developed a process for the production of high silica mordenites suitable for use as a solid acid catalyst in the low polymerization of isobutene.

According to the present invention there is provided a process for the selective low polymerization of isobutene which comprises contacting an isobutene-containing hydrocarbon mixture with a solid acid catalyst having a solid acid content of 0.05 to 0.25 mmol/g solid acid catalyst, represented by the adsorption of pyridine, the solid acid catalyst being a high silica mordenite having a $iO_2/Al_2O_3$ mole ratio of 50 to 200 and being obtained by subjecting a hydrogen ion exchanged form of mordenite or a precursor thereof to a hydrothermal treatment under a partial pressure of steam of 1 to 60% at a temperature in the range of from 600 to 1000°C and then contacting it with an acid.

We have made various efforts to develop a catalyst capable of selectively polymerizing only isobutene without exhibiting activity in the isomerization of 1-butene to 2-butene, and consequently, have reached the present invention. We have also succeeded in effectively removing isobutene from 1-butene without loss of the 1-butene selectively polymerizing isobutene contained as an impurity in 1-butene using the catalyst of the present invention, and separating and removing isobutene as low polymers.

The solid acid catalyst for use in the present invention, is a solid acid catalyst whose solid acid content, as determined by the pyridine adsorption method is in the range of from 0.05 to 0.25 millimol per 1g of solid acid catalyst. A solid acid catalyst having a pyridine adsorption consent of 0.05 to 0.25 mmol per 1g of solid acid catalyst is capable of selectively activating only the tertiary carbon atom of a lower olefin to give a carbonium ion.

The catalyst used in the present invention is a high silica content mordenite catalyst with an $SiO_2$ to $Al_2O_3$ mole ratio of 50 to 200 which is capable of selectively polymerizing only isobutene and has no activity in the isomerization of 1-butene to 2-butene.

The high silica mordenite catalyst whose solid acid content is controlled and which is used in the present invention is obtained by converting natural or synthetic mordenite having the following formula:

$$4Na_2O.4Al_2O_3.40SiO_2.24H_2O$$

into its hydrogen ion exchanged from a precursor thereof, subjecting it to a hydrothermal treatment at a partial pressure of steam of 1 to 60% at a temperature in the range of from 600 to 1000°C and then extraction of aluminium with a strong acid.

In general, in order to use such a mordenite effectively as a catalyst for the conversion of hydrocarbons, it is necessary to give it properties as a solid acid and to raise the reactivity thereof by cation exchange treatment wherein the majority of the cations contained therein are ion-exchanged with catalytically active metallic ions or hydrogen ions.

The conversion of mordenite into a hydrogen type mordenite by cation exchange can also be accomplished in conventional manner by ammonium ion exchange with ammonium chloride or by treatment with a relatively weak acid, since the mordenite itself has a relatively high silica to alumina ratio. Furthermore it is well known that on treatment with a relatively strong acid, not only a cation exchange reaction but also the removal of aluminium from the crystal lattice take place to further increase the silica to alumina

ratio in the mordenite. The thus treated mordenite, when used as a catalyst, has a high reactivity in hydrocarbon conversion reactions such as hydrocracking, thermal cracking, isomerization and disproportionation.

When mordenite is treated to increase the silica/alumina ratio by the prior art method, however, the solid acid quantity is too large or too small and control of the solid acid quantity is impossible.

We have developed a method of controlling the solid acid content of a crystalline aluminosilicate and consequently, have found a method of preparing a high silica mordenite having a silica/alumina ratio of 50 to 200 and a specified solid acid content or solid acid content or solid acid strength distribution, whereby the selective conversion of hydrocarbons is made possible. This method comprises subjecting mordenite to heat treatment in the presence of steam and then contacting the treated mordenite with a strong acid.

The raw material mordenite used herein can be any natural or synthetic mordenite. The mordenite is converted into a hydrogen ion form or hydrogen ion form precursor prior to hydrothermal treatment, but the raw mordenite can be combined with any metal ions, i.e., alkali metal ions or alkaline earth metal ions. The hydrogen exchanged form or hydrogen exchanged form precursor can be prepared by subjecting the above described raw material mordenite to ion exchange using an aqueous acid solution or ammonium salt solution. For example, hydrochloric acid, sulfuric acid or nitric acid may be used as the acid. The acid concentration is preferably at least 0.5 normal. The hydrogen ion exchange reaction is preferably carried out at a temperature of from room temperature to 100°C and heating is preferably employed to accelerate the conversion reaction.

The hydrothermal reaction is affected by a heat treatment in the presence of steam, during which the partial pressure of steam and the treatment temperature are so chosen as to control the silica/alumina ratio within the range of from 50 to 200 as well as the solid acid quantity.

For the purpose of adjusting the pyridine adsorption content to 0.05 to 0.25 mmol per 1g of high silica mordenite, the hydrothermal treatment is effected under a partial pressure of steam of 1 to 60%, preferably 5 to 40% at a temperature in the range of from 600 to 1000°C, preferably 650 to 750°C. At a temperature below this range, the solid acid content cannot be controlled. In the absence of steam, only a mordenite with a higher solid acid content can be obtained and if the steam partial pressure exceeds the above range, reactions other than the aluminium removing reaction tend to often occur, whereby the solid acid content is markedly decreased.

The hydrothermal treatment is generally carried out for a period of from 1 to 10 hours, preferably 2 to 5 hours with satisfactory results. Following the hydrothermal treatment, an acid-extraction treatment is carried out so as to remove $AlOH^{2+}$ dislocated from the mordenite skeletal structure by the hydrothermal treatment and being present as so-called nests or as aluminium atoms positioned in a relatively weak skeletal structure. Examples of the acid which may be used in the acid-extraction treatment are inorganic acids such as hydrochloric acid, nitric acid, sulfuric acid and phosphoric acid, and organic acids such as acetic acid, chloroacetic acid, trichloroacetic acid, citric acid, tartaric acid and oxalic acid. Of these acids, mineral acids such as hydrochloric acid, sulfuric acid and nitric acid are preferred. When using these acids in the extraction treatment, an acid concentration of at least 4N, preferably at least 6N is preferred. The acid-extraction treatment is preferably carried out at a temperature ranging from room temperature to 100°C.

The thus-extracted mordenite is preferably subjected to a calcining treatment under more moderate conditions than in the hydrothermal treatment, i.e., at a temperature of from 400 to 700°C, preferably 500 to 650°C to stabilize the unstable crystal state.

The thus obtained high silica mordenite with a pyridine adsorption content of 0.05 to 0.25 mmol per 1g of mordenite and a silica/alumina ratio of 50 to 200 can be used as the reaction catalyst of the present invention as it is or after forming.

The solid acid content or solid acid strength of the high silica content mordenite obtained in this way is determined by the adsorption-desorption method. That is to say, a basic material is caused to be adsorbed on solid acid sites, followed by raising the temperature, and the desorbed basic material is then determined, which corresponds to the adsorption quantity. The adsorption quantity of the basic material is directly related to the solid content and the temperature at which the basis material is desorbed is directly related to the solid acid strength. Thus, a relative solid acid content and solid acid strength distribution can be given.

Examples of basic materials which may be used in the adsorption-desorption method are ammonia, pyridine and n-butylamine. Pyridine is preferred, because the desorbed quantity can readily be determined by a hydrogen ion detecting method and pyridine is adsorbed on solid acid sites, i.e., Bronsted Acid sites as pyridinium ions and on Lewis Acid sites as coordinate bonded pyridine, so that the acid quantity can be determined relatively accurately.

According to the process of the present invention, a high silica mordenite with a pyridine adsorption content of 0.05 to 0.25 mmol per 1g of mordenite is obtained.

When the partial pressure of steam is increased and the treatment temperature is raised in the hydrothermal treatment, the solid acid content is decreased, whereas when the partial pressure of steam is decreased and the treatment temperature is lowered, the solid acid content is increased.

The solid acid catalyst, in particular, high silica mordenite used as a catalyst in the process of the present invention, has the property that deterioration of its catalytic performance scarcely takes place even when it is used for a long time, but its catalytic activity can be maintained for longer by previously contacting it with hydrocarbons at a temperature higher than the reaction temperature before it is used as a catalyst.

Any hydrocarbon can be used for the pretreatment, but unsaturated hydrocarbons such as olefins, diolefins and aromatic hydrocarbons, in particular, hydrocarbon mixtures are preferred as the raw material.

The pretreatment is preferably carried out in liquid phase or gaseous phase at a temperature higher than the reaction temperature of the main reaction (catalytic reaction of raw material hydrocarbons) and below 300°C for a period of time of from 10 minutes to 5 hours. More preferably, the pretreatment is carried out under the following conditions: liquid phase, reaction temperature 140 to 200°C, reaction time 0.5 to 2 hours, and liquid hourly space velocity (LHSV) 1 to 20 $hr^{-1}$.

As the hydrocarbon mixture used in the present invention, any hydrocarbon mixture containing isobutene and n-butene can be used, but on a commercial scale, it is preferable to use, for example, $C_4$ hydrocarbon mixtures obtained in the refining, cracking and reforming of oils, butane-butene fractions obtained by removing butadiene from $C_4$ fraction byproduced in the production of ethylene by the thermal cracking of oils or $C_4$ hydrocarbon mixtures obtained by the dehydrogenation of hydrocarbon mixtures containing n-butane and isobutane. The $C_4$ hydrocarbon mixtures can be used as they are.

These hydrocarbons generally include, in addition to n-butenes (1-butene, trans-2-butene, cis-2-butene) and isobutene, n-butane, isobutane, butadiene and trace amounts of $C_3$ hydrocarbons and $C_5$ hydrocarbons. The amounts of these components are not limited, but for producing high purity 1-butene, it is desirable to use hydrocarbon mixtures containing at least 20 mole % of n-butenes and 0.1 to 50 mole % of isobutene.

Low polymers of isobutene are obtained by contacting isobutene with a catalyst consisting of the hydrogen type crystalline aluminosilicate according to the present invention, during which the polymerization conditions are gaseous phase or liquid phase reaction, reaction temperature 20 to 180°C and reaction pressure of from 101 to 9800 KPa (atmospheric pressure to 100 $Kg/cm^2$) and the preferred polymerization conditions are liquid phase reaction, reaction temperature 60 to 149°C and a pressure capable of maintaining the reactants liquid, i.e., 980 to 4900 KPa (10 to 50 $Kg/cm^2$). For the liquid phase reaction, LHSV is 0.01 to 50 $hr^{-1}$, preferably 0.1 to 10 $hr^{-1}$.

According to the process of the present invention, isobutene contained in a raw material is substantially converted into low polymers, i.e., dimers and trimers and 1-butene is scarcely isomerized. The low polymers of isobutene are further separated and removed by any known methods. Thus, a substantially isobutene-free fraction can be obtained, from which 1-butene and/or 2-butene can further be recovered in a conventional manner.

Using the process of the present invention, isobutene contained in a raw material hydrocarbon mixture can be substantially converted into low polymers i.e., dimers and trimers, which can readily be separated from $C_4$ hydrocarbons, with substantially prevention of isomerization of 1-butene to 2-butene and loss of n-butenes, and accordingly, the isobutene can be completely removed by removing the low polymers. Thus, a high purity 1-butene and/or 2-butene which is substantially free from isobutene can be recovered from the fraction from which the polymers have been removed.

As is apparent from the foregoing illustration, the process of the present invention is available for the purification of 1-butene, since isobutene can be selectively polymerized without the isomerization of 1-butene and the low polymers is isobutene can readily be separated from the isobutene. One embodiment of this process is shown in the accompanying drawings in which:

Fig. 1 is a flow diagram to illustrate a process for the low polymerization of isobutene using a specific solid acid catalyst according to the present invention.

Fig. 2 and Fig. 3 are flow diagrams to illustrate processes for separating the components produced by the present invention.

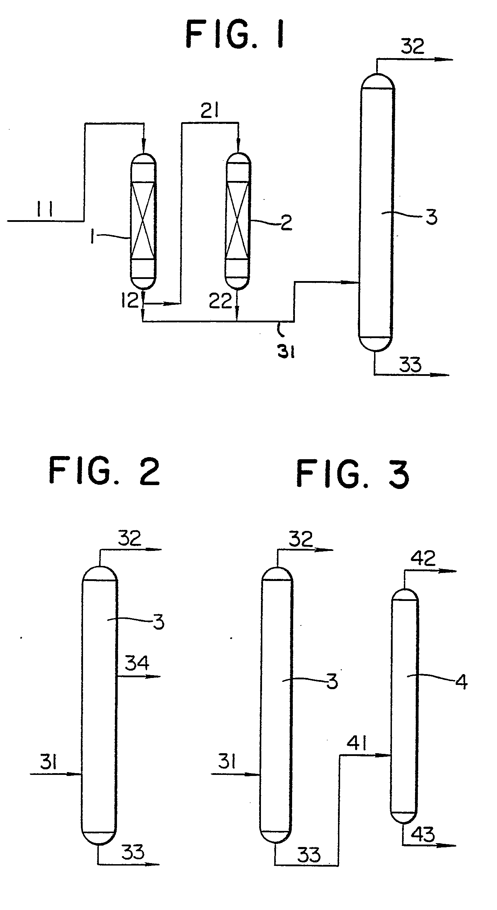

Referring to Fig. 1, a 1-butene fraction containing isobutene as an impurity is fed from pipe 11 to the isobutene polymerization column 1 packed with a catalyst whose solid acid content is controlled, where the isobutene contained in the 1-butene fraction is selectively low-polymerized, and then withdrawn via pipe 12. This fraction is further fed from pipe 21 to a second isobutene polymerization column 2 packed similarly with a catalyst whose solid acid content is controlled, wherein the residual isobutene is low-polymerized. The 1-butene fraction containing low polymers of isobutene is then fed through pipe 31 to distillation column 3, from which the low polymers of isobutene are withdrawn via pipe 33 and high purity 1-butene substantially free from isobutene is withdrawn via pipe 32 and recovered by distillation.

In Fig. 1, the embodiment of using two isobutene polymerization column has been illustrated, but when isobutene is contained in a trace amount, one isobutene polymerization column is sufficient to achieve the object and, if necessary, three or more columns can be used.

When 2-butene is contained in a 1-butene fraction, the separation of 1-butene and 2-butene is carried out by adjusting the operation conditions of distillation column 3 so that 2-butene is withdrawn from pipe 33 together with the low polymers of isobutene in Fig. 1. Moreover, as shown in Fig. 2, a distillation column 3 can be used which is designed so that 2-butene can be withdrawn

from pipe 34. In Fig. 1, a mixture of low polymers of isobutene and 2-butene is withdrawn through pipe 33 and then fed via pipe 41 to distillation column 4 as shown in Fig. 3, where 2-butene and the low polymers of isobutene are readily separated by distillation and respectively withdrawn from pipe 42 and pipe 43.

The following examples are given in order to illustrate the present invention in greater detail.

Example 1

Preparation of high silica mordenite catalyst

100g of 1/6 inch pellets of a commercially sold high crystalline sodium type mordenite (commercial name: Zeolan 900 Na manufactured by Norton & Co.) was immersed in 500 ml of 1 N HCl and stirred at 80°C for 1 hour. Then, the acid solution was removed by decantation and 500 ml of new 1 N HCl was added, followed by stirring at 80°C for 1 hour. After removing the hydrochloric acid solution, the reaction product was washed with warm water until no chloride ion was found and dried by hot air at 110°C to obtain a hydrogen ion exchanged type precursor of mordenite. The resulting hydrogen ion exchange type precursor was subjected to a hydrothermal treatment under a steam partial pressure of 30% of for 4 hours at 640°C, the temperature being gradually raised. After cooling, the precursor was subjected to a refluxing treatment at 90°C for 6 hours using 500 ml of 12 N HCl to extract aluminium. After removing the hydrochloric acid solution, the product was washed with warm water until no chloride ion was found and then dried by hot air at 110°C. After air drying, the product was calcined at 650°C for 3 hours in a muffle furnace to thus obtain a high silica content mordenite with a composition and silica/alumina ratio as shown in Table 1.

The thus obtained high silica mordenite was ground in a size of 30 to 100 mesh and calcined at 500°C for 1 hour to remove adsorbed components. 0.075g of the mordenite was ground in a size of 30 to 100 mesh and calcined at 500°C for 1 hour to remove adsorbed components. 0.075 g of the mordenite was taken by precisely weighing and charged in a reactor. On the other hand, a pyridine-charged bubbler was immersed in a water bath held at a constant temperature, i.e., 15.5°C. Nitrogen gas was bubbled in the pyridine and fed to the mordenite catalyst charged in the reactor, where pyridine was caused to adsorb on the catalyst at room temperature.

With flowing nitrogen gas, the reactor was heated gradually to 300°C and held at 300°C until the pyrdine adsorbed physically be desorbed. When the desorption of the pyridine was not found by gas chromatography, the temperature of the reactor was raised at a rate of 10°C/min from 300°C to 950°C and during the same time, the desorbed pyridine was determined by gas chromatography. The quantity of the desorbed pyridine is in proportion to the solid acid quantity of the mordenite, as shown in Table 1.

Catalytic reaction

A $C_4$ hydrocarbon consisting of 26.2 mole % of butane, 1.3 mole % of isobutene, 7.5 mole % of 1-butene and 65 mole % of 2-butene was fed to a cylindrical reactor packed with the above described catalyst and subjected to catalytic reaction in liquid phase at a reaction temperature of 80°C, reaction pressure of 3430 KPa (35 $Kg/cm^2$) (compressed by nitrogen gas) and LHSV of 3.0 $hr^{-1}$.

After 16 hours from the start of reaction, the hydrocarbon mixture taken at the outlet of the reactor was analysed thus obtaining results shown in Table 1. With the passage of the reaction time, conversion of the isobutene was traced to seek degradation coefficient $\alpha$ from $k=k_oe\alpha t$ where k is an isobutene dimerization rate constant at time t, $k_o$ is an initial rate constant obtained by extrapolation and is a degradation coefficient. The results are shown in Table 1.

Comparative Example 1

The hydrogen ion exchanged type precursor obtained in Example 1 was calcined at 650°C for 4 hours using a muffle furnace, and then subjected to extraction with hydrochloric acid and calcination in an analogous manner to Example 1 to obtain a mordenite catalyst with a pyridine adsorption quantity, composition and silica/alumina ratio as shown in Table 1. Using this catalyst, a catalytic reaction was carried out in an analogous manner to Example 1, thus obtaining results shown in Table 1.

Comparative Example 2

A mordenite catalyst was prepared in an analogous manner to Example 1 except using a steam partial pressure of 100%, treatment temperature of 700°C and treatment time of 3 hours in the hydrothermal treatment of Example 1, and then subjected to catalytic reaction, thus obtaining results shown in Table 1.

Comparative Example 3

A mordenite catalyst was prepared in an analogous manner to Example 1 except using a steam partial pressure of 65%, treatment temperature of 650°C and treatment time of 3 hours in the hydrothermal treatment of Example 1 and then subjected to catalytic reaction to thus obtain results as shown in Table 1.

Example 2

Example 1 was repeated except using nitric acid instead of the hydrochloric acid in the acid treatment of Example 1 to obtain results shown in Table 1.

Example 3

Example 1 was repeated except using sulfuric acid instead of the hydrochloric acid in the acid treatment of Example 1 to thus obtain results shown in Table 1.

Example 4

Preparation of catalyst

67g of aluminium sulfate, 19.7g of conc. $H_2SO_4$ and 140g of sodium chloride were dissolved in 1500g of pure water, to which 490g of water glass (JIS No. 3) was then added to form an aqueous reaction mixture with a composition of $2.5Na_2O.Al_2O_3.23SiO_2.99H_2O$. This reaction mixture was aged at room temperature for about 1 hour, charged in an autoclave, rapidly heated and held at 180°C for 20 hours. The thus resulting solid product was cooled to room temperature, filtered, washed adequately with water and dried at 110°C. A part of the product was calcined at 700°C in the air and then subjected to adsorption of water at room temperature and to chemical analysis. The resulting synthetic mordenite had the following composition:

| | |
|---|---|
| Ignition Loss at 800°C | 10.0% by weight |
| $SiO_2$ | 79.1% by weight |
| $Al_2O_3$ | 5.96% by weight |
| $Na_2O$ | 3.51% by weight |
| $SiO_2/Al_2O_3$ (mole ratio) | 22.6% by weight |

In addition, it was found from the spacing in X-ray powder diffraction analysis that the product had the crystalline structure of mordenite.

100g of the resulting synthetic mordenite was processed in the same manner as in Example 1 to thus obtain a high silica mordenite with a composition and pyridine adsorption quantity as shown in the following:

Composition

| | |
|---|---|
| Ignition Loss at 800°C | 3.2% by weight |
| $SiO_2$ | 95.1% by weight |
| $Al_2O_3$ | 1.22% by weight |
| $Na_2O$ | 0.11% by weight |
| $SiO_2/Al_2O_3$ (mole ratio) | 133 % by weight |
| Pyridine Adsorption Quantity (mmol/g. mordenite) | |
| Total Pyridine Adsorbed | 0.108% by weight |

Catalytic Reaction

Using this catalyst, a catalytic reaction was carried out in an analogous manner to Example 1, thus obtaining results shown in Table 1.

Example 5

Example 1 was repeated except using a steam partial pressure of 5%, treatment temperature of 700°C and treatment time of 3 hours in the hydrothermal treatment of Example 1 to obtain results shown in Table 1.

Example 6

Example 1 was repeated except using a steam partial pressure of 20%, treatment temperature of 700°C and treatment time of 3 hours in the hydrothermal treatment of Example 1 to thus obtain results shown in Table 1.

Comparative Example 4

The hydrogen ion exchanged type precursor obtained in Example 1 was calcined at 600°C for 3 hours in a muffle furnace to obtain a hydrogen ion exchange type mordenite. Using this catalyst, a catalytic reaction was carried out in an analogous manner to Example 1, thus obtaining results shown in Table 1.

Example 7

A part of the catalyst of Example 5 was retreated using the same hydrothermal conditions as those of Example 5. The results were shown in Table 1.

Example 8

Example 1 was repeated except using a steam partial pressure of 45%, treatment temperature of 660°C and treatment time of 4 hours in the hydrothermal treatment of Example 1 to obtain results shown in Table 1.

Example 9

Example 1 was repeated except using a steam partial pressure of 20%, treatment temperature of 630°C and treatment time 3 hours in the hydrothermal treatment of Example 1 to obtain results shown in Table 1.

Example 10

Example 4 was repeated except using a steam partial pressure of 45%, treatment temperature of 660°C and treatment time 4 hours in the hydrothermal treatment of Example 4 to obtain results shown in Table 1.

TABLE 1

| | Mordenite Catalyst | | | | | | | | | |
|---|---|---|---|---|---|---|---|---|---|---|
| | | Composition | | | | | | | | |
| Example No. | Pyridine Adsorption Quantity (mmol/g. mordenite) | Ignition Loss at 800°C (wt %) | $SiO_2$ (wt %) | $Al_2O_3$ (wt %) | $Na_2O$ (wt %) | $SiO_2/Al_2O_3$ (wt %) | Isobutene Conversion (mole %) | n-Butene Loss (mole %) | $k_o$ | $\alpha$ (l/hr) |
| 1 | 0.113 | 5.3 | 92.7 | 1.75 | 0.17 | 90 | 89.3 | 3.2 | 9.8 | 0.0032 |
| 2 | 0.112 | 4.4 | 93.8 | 1.50 | 0.15 | 106 | 91.0 | 4.3 | 11.5 | 0.0019 |
| 3 | 0.118 | 4.5 | 93.1 | 1.79 | 0.12 | 96 | 86.7 | 2.9 | 8.0 | 0.0041 |
| 4 | 0.108 | 3.2 | 95.1 | 1.22 | 0.11 | 133 | 91.2 | 3.8 | 12.0 | 0.0033 |
| 5 | 0.121 | 5.5 | 92.0 | 1.81 | 0.16 | 86 | 88.1 | 4.2 | 8.8 | 0.0029 |
| 6 | 0.111 | 4.3 | 94.1 | 1.50 | 0.13 | 107 | 92.1 | 5.1 | 13.5 | 0.0041 |
| 7 | 0.119 | 5.0 | 93.6 | 1.33 | 0.07 | 120 | 90.3 | 2.5 | 12.9 | 0.0018 |
| 8 | 0.098 | 2.8 | 96.0 | 1.09 | 0.14 | 152 | 85.3 | 2.9 | 7.6 | 0.0035 |
| 9 | 0.230 | 5.9 | 91.9 | 1.98 | 0.16 | 79 | 93.1 | 5.3 | 14.0 | 0.0053 |
| 10 | 0.095 | 2.9 | 95.9 | 0.92 | 0.14 | 177 | 81.2 | 2.8 | 5.1 | 0.0037 |
| Comparison | | | | | | | | | | |
| 1 | 0.360 | 8.6 | 86.5 | 4.1 | 0.18 | 36 | 48.0 | 12.3 | 1.2 | 0.0052 |
| 2 | 0.041 | 3.5 | 95.2 | 1.20 | 0.11 | 135 | 26.0 | 2.1 | 0.3 | 0.0081 |
| 3 | 0.042 | 3.9 | 94.4 | 1.51 | 0.14 | 106 | 68.0 | 25.6 | 3.5 | 0.0076 |
| 4 | 0.544 | 6.8 | 80.5 | 10.0 | 1.06 | 13.7 | 76.5 | 38.1 | 16.0 | 0.190 |

**Claims**

1. A process for the selective polymerization of isobutene which comprises contacting an isobutene-containing hydrocarbon mixture with a solid acid catalyst characterized in that the solid acid catalyst has a solid acid content of 0.05 to 0.25 mmol/g. solid acid catalyst, represented by the adsorption of pyridine, the solid acid catalyst being a high silica mordenite having a $SiO_2/Al_2O_3$ mole ratio of 50 to 200 and being obtained by subjecting a hydrogen ion exchanged form of a mordenite or a precursor thereof to a hydrothermal treatment under a partial pressure of steam of 1 to 60% at a temperature in the range of from 600 to 1000°C and then contacting it with an acid.

2. A process as claimed in Claim 1 wherein the acid is hydrochloric acid, nitric acid, sulfuric acid, phosphoric acid, acetic acid, chloroacetic acid, trichloroacetic acid, citric acid, tartaric acid or oxalic acid.

3. A process as claimed in Claim 2 wherein the acid has a concentration of at least 4 normal.

4. A process as claimed in Claim 2 or Claim 3 wherein the contacting is carried out at a temperature in the range of from room temperature to 100°C.

5. A process as claimed in Claim 1 wherein the mordenite contacted with an acid is further subjected to a stabilizing treatment at a temperature in the range of from 400 to 700°C.

6. A process as claimed in any one of the preceding Claims wherein the solid acid catalyst is subjected to a pretreatment by previously contacting it with at least one hydrocarbon at a temperature higher than the temperature at which it is contacted with the isobutene-containing hydrocarbon mixture.

7. A process as claimed in Claim 6 wherein the pretreatment is carried out in the liquid phase or gaseous phase at a temperature higher than the contacting temperature and below 300°C.

8. A process as claimed in Claim 7 wherein the pretreatment is carried out in the liquid phase at a temperature in the range of from 140 to 200°C for 0.5 to 2 hours with a liquid hourly space velocity of 1 to 20 $hr^{-1}$.

9. A process as claimed in any one of the preceding Claims wherein the isobutene-containing hydrocarbon mixture is a hydrocarbon mixture containing at least 20 mole % of n-butenes and 0.1 to 50 mole % of isobutene.

10. A process as claimed in any one of the preceding Claims wherein the contacting is carried out in the liquid phase or gaseous phase at a temperature in the range of from 20 to 180°C under a pressure of from 101 to 9800 KPa (atmospheric pressure to 100 $Kg/cm^2$).

11. A process as claimed in Claim 10 wherein the contacting is carried out in the liquid phase at a temperature in the range of from 60 to 140°C under a pressure of 980 to 4900 KPa (10 to 50 $Kg/cm^2$) with a liquid hourly space velocity of 0.01 to 50 $hr^{-1}$.

12. A method of purifying 1-butene containing isobutene which comprises contacting an isobutene-containing hydrocarbon mixture with a solid acid catalyst having a solid acid content of 0.05 to 0.25 mmol/g. solid acid catalyst, represented by the adsorption of pyridine, the solid acid catalyst being a high silica mordenite having a $SiO_2/Al_2O_3$ mole ratio of 50 to 200 and being obtained by subjecting a hydrogen ion exchanged form of mordenite or a precursor thereof to a hydrothermal treatment under a partial pressure of steam of 1 to 60% at a temperature in the range of from 600 to 1000°C and then contacting it with an acid, thereby selectively low-polymerizing isobutene and then separating the low polymers of isobutene from the 1-butene.

**Patentansprüche**

1. Verfahren zur selektiven Polymerisation von Isobuten durch Inberührungbringen eines isobutenhaltigen Kohlenwasserstoffgemischs mit einem festen Säurekatalysator, dadurch gekennzeichnet, daß der feste Säurekatalysator einen durch Pyridinadsorption ermittelten Gehalt an fester Säure von 0,05 bis 0,25 mMol/g fester Säurekatalysator aufweist und aus einem einen hohen $SiO_2$-Gehalt aufweisenden Mordenit eines Molverhältnisses $SiO_2/Al_2O_3$ von 50 bis 200, der durch hydrothermale Behandlung eines durch Ionenaustausch in eine wasserstoffionenhaltige Mordenit- oder Mordenitvorläuferform umgewandelten Mordenits oder Mordenitvorläufers unter einem Dampfpartialdruck von 1 bis 60% bei einer Temperatur im Bereich von 600 bis 1000°C und anschließendes Inberührungbringen mit einer Säure hergestellt wurde, besteht.

2. Verfahren nach Anspruch 1, dadurch gekennzeichnet, daß die Säure aus Chlorwasserstoff-, Salpeter-, Schwefel-, Phosphor-, Essig-, Chloressig-, Trichloressig-, Zitronen-, Wein- oder Oxalsaure besteht.

3. Verfahren nach Anspruch 2, dadurch gekennzeichnet, daß die Säure eine Konzentration von mindestens 4 normal aufweist.

4. Verfahren nach Ansprüchen 2 oder 3, dadurch gekennzeichnet, daß das Inberührungbringen bei einer Temperatur im Bereich von Raumtemperatur bis 100°C erfolgt.

5. Verfahren nach Ansprüch 1, dadurch gekennzeichnet, daß der mit einer Säure in Berührung gebrachte Mordenit zusätzlich bei einer Temperatur im Bereich von 400 bis 700°C einer Stabilisierungsbehandlung unterworfen wird.

6. Verfahren nach einem der vorhergehenden Ansprüche, dadurch gekennzeichnet, daß der feste Säurekatalysator durch vorheriges Inberührungbringen mit mindestens einem Kohlenwasserstoff bei einer Temperatur oberhalb der Temperatur, bei der er mit dem isobutenhaltigen Kohlenwasserstoffgemisch in Berührung gebracht wird, einer Vorbehandlung unterworfen wird.

7. Verfahren nach Anspruch 6, dadurch gekennzeichnet, daß die Vorbehandlung in flüssiger oder

gasförmiger Phase bei einer Temperatur oberhalb der Temperatur des Inberührungbringens und unterhalb von 300°C erfolgt.

8. Verfahren nach Anspruch 7, dadurch gekennzeichnet, daß die Vorbehandlung in flüssiger Phase während 0,5 bis 2 h bei einer Temperatur im Bereich von 140 bis 200°C und bei einer stündlichen Flüssigkeitsraumgeschwindigkeit von 1 bis 20 $h^{-1}$ erfolgt.

9. Verfahren nach einem der vorhergehenden Ansprüche, dadurch gekennzeichnet, daß das isobutenhaltige Kohlenwasserstoffgemisch aus einem Kohlenwasserstoffgemisch mit mindestens 20 Mol% N-Butenen und 0,1 bis 50 Mol% Isobuten besteht.

10. Verfahren nach einem der vorhergehenden Ansprüche, dadurch gekennzeichnet, daß das Inberührungbringen in flüssiger oder gasförmiger Phase bei einer Temperatur im Bereich von 20 bis 180°C unter einem Druck von 101 bis 9800 KPa (Atmosphärendruck bis 100 $kg/cm^2$) erfolgt.

11. Verfahren nach Anspruch 10, dadurch gekennzeichnet, daß das Inberührungbringen in flüssiger Phase bei einer Temperatur im Bereich von 60 bis 140°C unter einem Druck von 980 bis 4900 KPa (10 bis 50 $kg/cm^2$) bei einer stündlichen Flüssigkeitsraumgeschindigkeit von 0,01 bis 50 $h^{-1}$ erfolgt.

12. Verfahren zum Reinigen von 1-Buten enthaltendem Isobuten, dadurch gekennzeichnet, daß man zur selektiven Niedrigpolymerisation von Isobuten ein isobutenhaltiges Kohlenwasserstoffgemisch mit einem festen Säurekatalysator eines durch Pyridinadsorption bestimmbaren Gehalts an fester Säure von 0,05 bis 0,25 mMol/g fester Säurekatalysator, der aus einem einen hohen $SiO_2$-Gehalt aufweisenden Mordenit eines Molverhältnisses $SiO_2/Al_2O_3$ von 50 bis 200 besteht und durch eine hydrothermale Behandlung eines durch Ionenaustausch in eine wasserstoffionenhaltige Mordenit- oder Mordenitvorläuferform umgewandelten Mordenits oder Mordenitvorläufers unter einem Dampfpartialdruck von 1 bis 60% bei einer Temperatur im Bereich von 600 bis 1000°C und anschließendes inberührungbringen mit einer Säure hergestellt wurde, in Berührung bringt und danach die niedrigen Isobutenpolymerisate von dem 1-Buten abtrennt.

**Revendications**

1. Procédé de polymérisation sélective de l'isobutène qui comprend une étape consistant à mettre en contact un mélange d'hydrocarbures contenant de l'isobutène avec un catalyseur acide solide, caractérisé en ce que le catalysateur acide solide a une teneur en acide solide de 0,05 à 0,25 mmole/g de catalyseur acide solide, teneur déterminée par l'adsorption de pyridine, le catalyseur acide solide étant une mordénite à haute teneur en silice et de rapport molaire $SiO_2/Al_2O_3$ de 50 à 200, et obtenu en soumettant une forme de mordénite avant subi un échange d'ions hydrogène ou un précurseur de celle-ci à un traitement hydrothermique à une pression partielle de vapeur de 1 à 60% et à une température située dans la plage de 600 à 1000°C, puis en le mettant en contact avec un acide.

2. Procédé selon la revendication 1 dans lequel l'acide est de l'acide chlorhydrique, nitrique, sulfurique, phosphorique, acétique, chloroacétique, trichloroacétique, citrique, tartrique, ou oxalique.

3. Procédé selon la revendication 2 où l'acide a une concentration d'au moins 4N.

4. Procédé selon la revendication 2 ou 3 dans lequel le contact s'effectue dans une plage de température allant de la température ambiante à 100°C.

5. Procédé selon la revendication 1 dans lequel la mordénite mise en contact avec un acide est en outre soumise à un traitement de stabilisation à une température située dans la plage allant de 400 à 700°C.

6. Procédé selon l'une quelconque des revendications précédentes dans lequel le catalyseur acide solide est soumis à un traitement préalable consistant à le mettre en contact avec au moins un hydrocarbure à une température supérieure à la température de contact avec le mélange d'hydrocarbures contenant de l'isobutène.

7. Procédé selon la revendication 6 où le traitement préalable est effectué en phase liquide ou gazeuse à une température supérieure à la température de contact, et inférieure à 300°C.

8. Procédé selon la revendication 7 où le traitement préalable est effectué en phase liquide à une température située dans la plage allant de 140 à 200°C pendant 0,5 à 2 heures avec une vitesse spatiale horaire de liquide de 1 à 20 $heures^{-1}$.

9. Procédé selon l'une quelconque des revendications précédentes dans lequel la mélange d'hydrocarbures contenant de l'isobutène est un mélange d'hydrocarbures contenant au moins 20% en moles de n-butènes et 0,1 à 50% en moles d'isobutène.

10. Procédé selon l'une quelconque des revendications précédentes dans lequel le contact est effectué en phase liquide ou gazeuse à une température située dans une plage allant de 20 à 180°C, à une pression de 101—9800 KPa (pression atmosphérique — 100 $Kg/cm^2$).

11. Procédé selon la revendication 10 dans lequel le contact est effectué en phase liquide à une température située dans la plage allant de 60 à 140°C, à une pression de 980—4900 KPa (10—50 $Kg/cm^2$), avec une vitesse spatiale horaire de liquide de 0,01 à 50 $heures^{-1}$.

12. Méthode pour purifier du 1-butène contenant de l'isobutène qui comprend une étape consistant à mettre en contact un mélange d'hydrocarbures contenant de l'isobutène avec un catalyseur acide solide ayant une teneur en acide solide de 0,05 à 0,25 mmole/g de catalyseur acide solide, teneur déterminée par l'adsorption de pyridine, le catalyseur acid solide étant une mordénite à haute teneur en silice et de rapport molaire $SiO_2/Al_2O_3$ de 50 à 200, et obtenu en

soumettant une forme de mordénite ayant subi un échange d'ions hydrogène ou un précurseur de celle-ci à un traitement hydrothermique à une pression partielle de vapeur de 1 à 60%, et à une température située dans la plage allant de 600 à 1000°C, puis en le mettant en contact avec un acide, ce dont il résulte la polymérisation sélective et à faible degré de l'isobutène, et en séparant ensuite les bas polymères d'isobutène du 1-butene.

FIG. 1

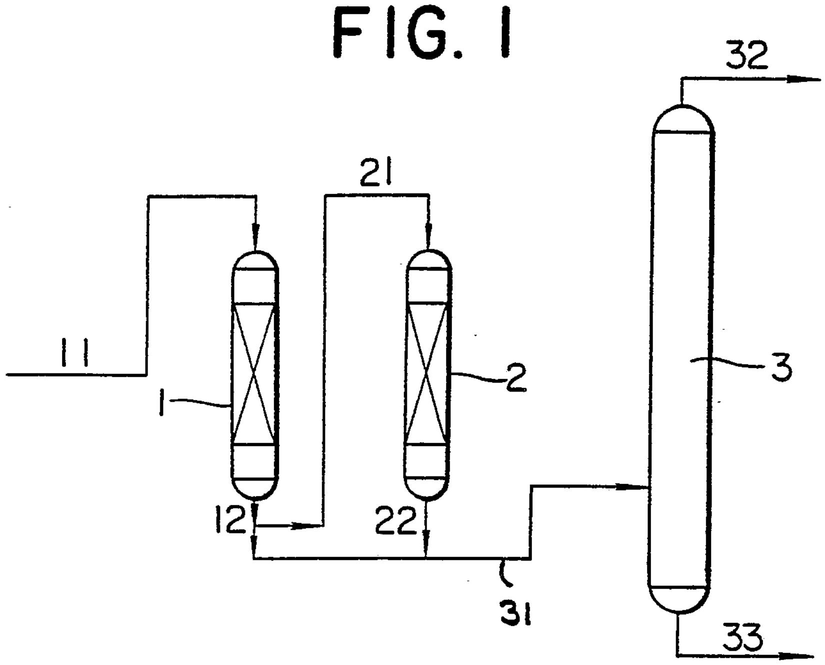


FIG. 2

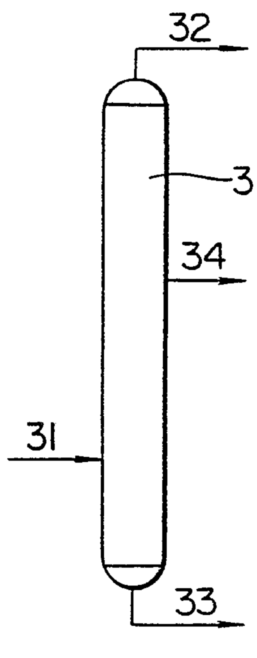


FIG. 3